## Europäisches Patentamt

## European Patent Office

## Office européen des brevets

(11) Veröffentlichungsnummer: **0 359 140**
**A2**

## (12) EUROPÄISCHE PATENTANMELDUNG

(21) Anmeldenummer: **89116596.1**

(22) Anmeldetag: **08.09.89**

(51) Int. Cl.5 **G01N 19/04**

(30) Priorität: **14.09.88 DE 8811632 U**

(43) Veröffentlichungstag der Anmeldung:
**21.03.90 Patentblatt 90/12**

(84) Benannte Vertragsstaaten:
**AT BE CH DE ES FR GB GR IT LI LU NL SE**

(71) Anmelder: **Freundl, Josef**
**Werner-v. Siemensstrasse 3**
**D-3015 Wennigsen(DE)**

(72) Erfinder: **Freundl, Josef**
**Werner-v. Siemensstrasse 3**
**D-3015 Wennigsen(DE)**

(74) Vertreter: **Arendt, Helmut, Dipl.-Ing.**
**Hubertusstrasse 2**
**D-3000 Hannover 1(DE)**

(54) **Haftzugprüfgerät.**

(57) Für die Ermittlung der Haftzugfestigkeit verschiedener Materialien soll ein von elektrischen Netzanschlüssen unabhängiger Einsatz ermöglicht werden. Zu diesem Zweck ist der Zugaufnehmer des Gerätes über ein Schneckengetriebe mit einer netzunabhängigen Antriebseinrichtung verbunden. Diese kann aus einer Handkurbel oder einem elektrischen, von einem Akkumulator gespeisten Servomotor bestehen.

EP 0 359 140 A2

Fig 1

## Haftzugprüfgerät

Die Erfindung betrifft ein Haftzugprüfgerät zur Ermittlung der Haftzugfestigkeit von Beton, Estrich, Mörtel. Bitumen und dergleichen sowie von Beschichtungen aus Natur- und Kunststoffen, bestehend aus einem Traggestell für einen Zugaufnehmer, an dessen dem Prüfobjekt zugewandten Ende ein Prüfstempel befestigt ist und mechanischen Antriebselementen zur Erzeugung der auf das zu prüfende Objekt zu übertragenden Zugkraft.

Bekannt sind Haftzugprüfgeräte mit einem elektrohydraulischen Antrieb zur Erzeugung der für einen Prüfvorgang notwendigen Zugkraft. Für die Prüfungen gelten Vorschriften, nach denen die in N.sec gemessenen Belastungszunahmen genau einzuhalten sind. Die Belastungszunahmen haben einer Geraden zu folgen, deren Steigungswinkel konstant ist. Diese Gerade als zeitabhängige Spannungsfunktion $\sigma$ (t) wird allgemein als "Rampe" bezeichnet.

Durch den elektrohydraulischen Antrieb ist es möglich, die Belastungszunahme sehr genau dem Rampenverlauf folgen zu lassen. Nachteilig ist die Abhängigkeit von einem Netzanschluß. Um eine Prüfung durchführen zu können, sind deshalb häufig lange Anschlußleitungen zu verlegen. Der Einsatz des Gerätes ist dadurch örtlich begrenzt.

Der Neuerung liegt die Aufgabe zugrunde, ein Gerät nach dem eingangs genannten Gattungsbegriff so auszubilden, daß ein von elektrischen Netzanschlüssen völlig unabhängiger Einsatz ermöglicht wird. Die neuerungsgemäße Lösung zeichnet sich dadurch aus, daß der Zugaufnehmer über ein Schneckengetriebe mit einer von einem Netzanschluß unabhängigen Antriebsvorrichtung verbunden ist. Bevorzugt eignen sich hierfür sowohl eine Handkurbel als auch ein von einem Akkumulator gespeister elektrischer Servomotor. Hierbei kann der Servomotor mit einem zugehörigen Untersetzungsgetriebe als gegen die Handkurbel austauschbare, von einem Gehäuse halbseitig umschlossene Einheit ausgebildet sein, wobei die Antriebswelle des Getrieberades des Untersetzungsgetriebes drehmomentübertragend in die Antriebsschnecke greift.

Weitere den Erfindungsgegenstand vorteilhaft gestaltende Merkmale sind in den Unteransprüchen angegeben.

Der Einsatz des Prüfgerätes unabhängig von einem Netzanschluß ist für die Praxis von großer Bedeutung. Trotz der Netzunabhängigkeit ist die Möglichkeit gegeben, die Belastungszunahme der vorgegebenen Rampe ohne erkennbare Abweichungen folgen zu lassen. Die Prüfungsvorschriften können dadurch mit höchster Genauigkeit eingehalten werden.

In der Zeichnung ist ein Ausführungsbeispiel der Erfindung mit zwei alternativen Antriebsvorrichtungen schematisch dargestellt und nachstehend erläutert.

Es zeigen:

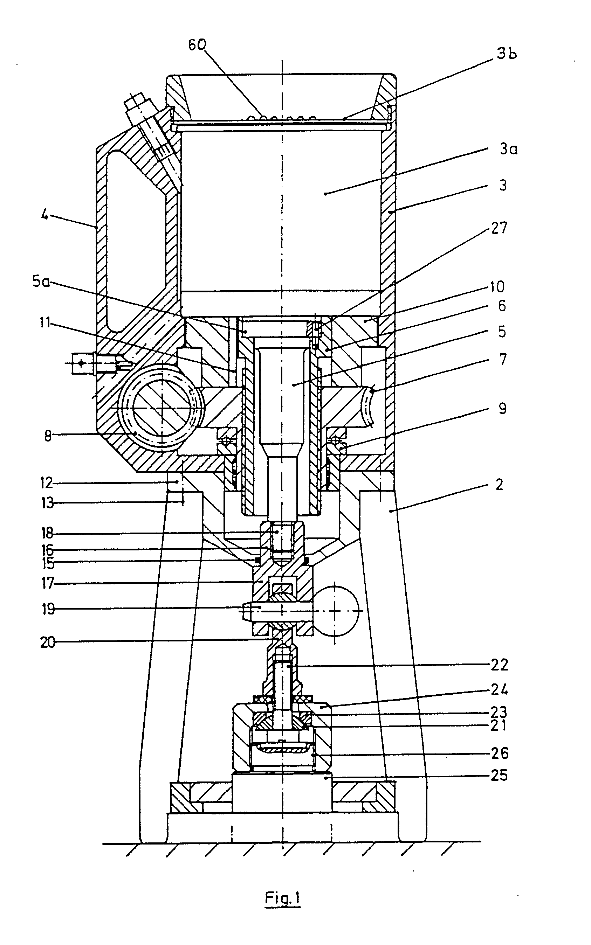

Fig. 1 die Seitenansicht eines Haftzugprüfgerätes zum Teil im Schnitt,

Fig. 2 die Draufsicht auf das Haftzugprüferät nach Fig. 1 mit einem Servomotor,

Fig. 3 die Draufsicht auf das Haftzugprüfgerät nach Fig. 1 mit einer Handkurbel,

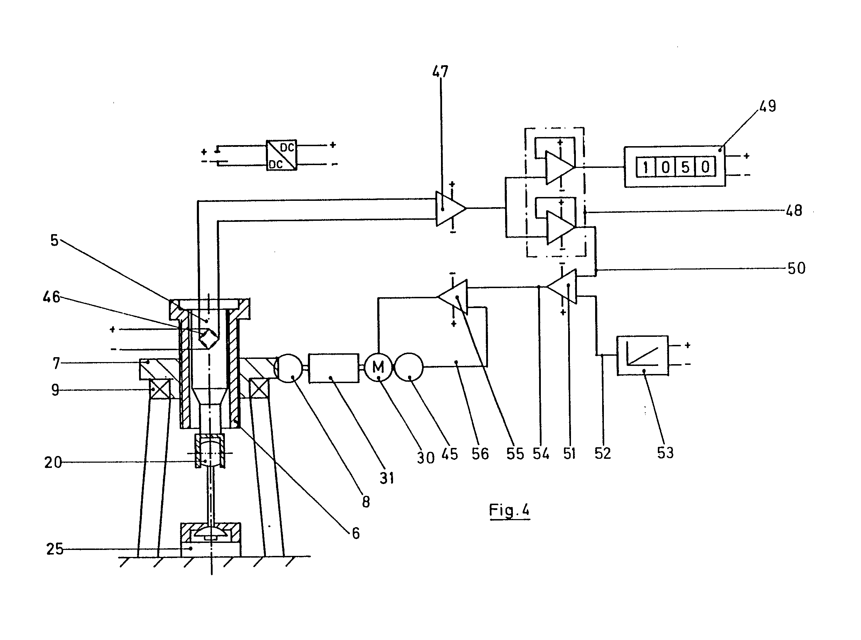

Fig. 4 eine Prinzipskizze des Erfindungsgegenstandes zusammen mit einem Blockschaltbild des Regelkreises und

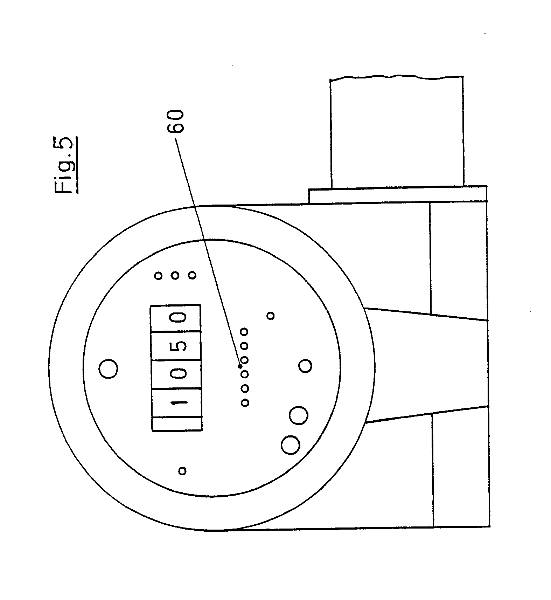

Fig. 5 die Draufsicht auf die Frontplatte mit den Leuchtdioden zur Kontrolle der Belastungsgeschwindigkeit.

Der räumliche Aufbau des Haftzugprüfgerätes 1 zeigt ein dreibeiniges Traggestell 2 und ein mit diesem verschraubtes Gehäuse 3 mit einem Handgriff 4. Innerhalb des Gehäuses sind der Zugaufnehmer 5, eine hülsenförmige Gewindespindel 6, ein Schneckenrad 7 mit der Gewindeschnecke 8 und das Drucklager 9 für die Aufnahme des Auflagerdrucks des Schneckenrades angeordnet. Ein hohlzylindrischer Einsatz 10 sorgt für die Führung der Gewindespindel 6 in ihrem oberen Teil. Gleichzeitig wird durch eine an dem Einsatz 10 befestigte Führungsschiene 11 eine Verdrehsicherheit der Gewindespindel erreicht. Der Einsatz 10 ist mit einem Außengewinde versehen, mit dem er in das Innengewinde des Gehäuses 3 eingeschraubt ist. Mit 3a ist ein Gehäuseeinsatz zur Aufnahme der elektronischen Schaltung bezeichnet.

Das dreibeinige Traggestell 2 ist mit einem Flanschring 12 versehen, an welchem die Beine des Traggestells durch Schrauben 13 befestigt sind. Eine Verschweißung ist alternativ möglich. An den Flanschring schließt sich ein nach unten gezogener, in seinem Endteil konisch geformter Boden an, der das Gehäuse nach unten mit Hilfe eines Dichtungsringes 15 abschließt. Dieser Ring umfaßt den Schaft 16 einer Befestigungsgabel 17. In den Schaft 16 greift ein Gewindeansatz 18 des Zugaufnehmers 5. Die Befestigungsgabel 17 trägt einen Gelenkkopf 20, in welchen ein zylindrischer Schraubansatz 22 einer Kugelkalotte 21 greift. Der Gelenkkopf 20 wird in der Befestigungsgabel durch einen horizontalen Befestigungsbolzen 19 gehalten. Die Kugelkalotte 21 trägt über einen Druck ring 23 aus Lagermetall eine Schraubmutter 24, in welche der Prüfstempel 25 mit einem Schraubansatz 26 greift.

Der Zugaufnehmer 5 ist mit einem Flansch 5a in der Gewindespindel 6 hängend gelagert und durch eine Stiftschraube 27 gegen Verdrehung gesichert.

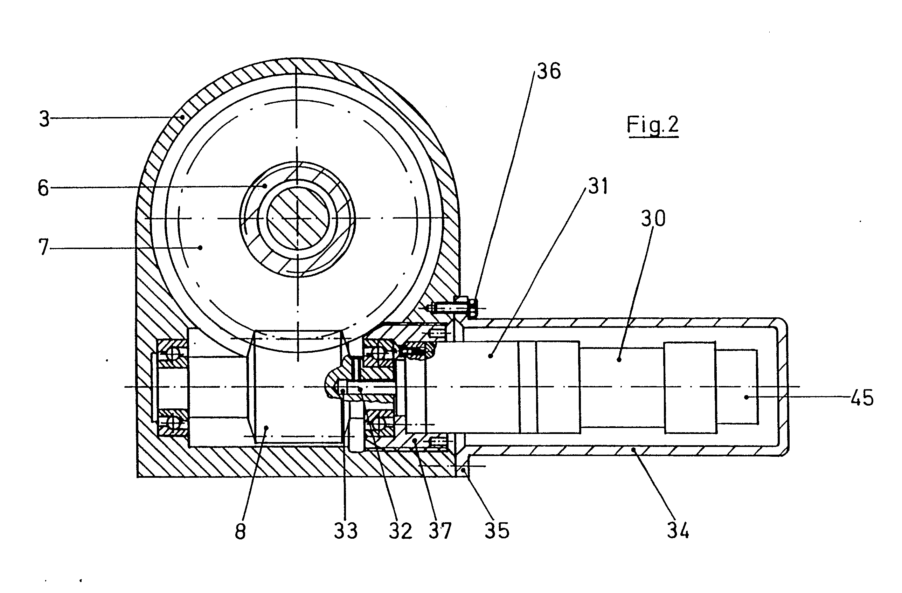

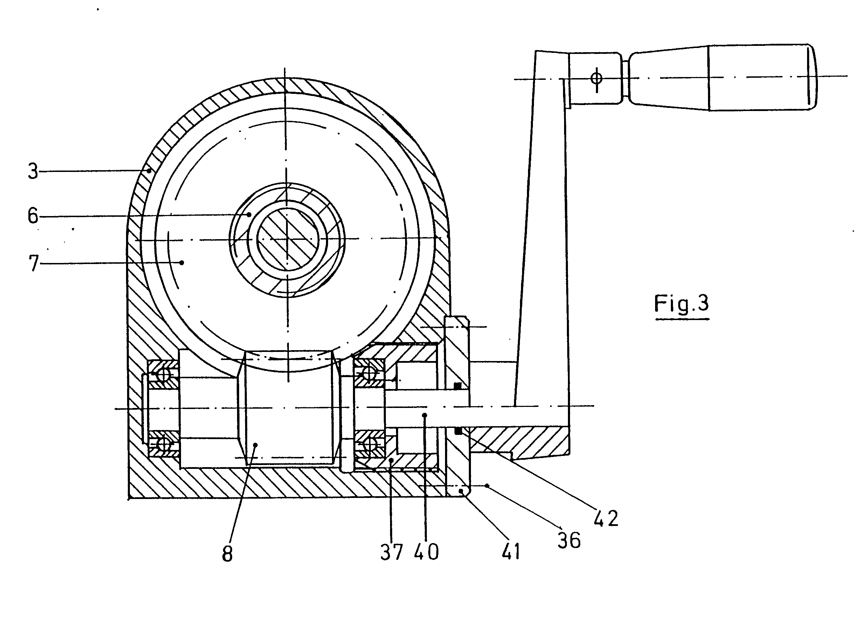

Durch die Kugelkalotte 21 wird der Prüfstempel im Verbindungsbereich mit dem Zugaufnehmer kardanisch aufgehängt, so daß ein Schiefzug durch einen nicht immer zu vermeidenden ungleichmäßigen Kleberauftrag vermieden und dadurch die Genauigkeit der Prüfung zusätzlich erhöht wird. Wie insbesondere den Fig. 2 und 3 zu entnehmen ist, können die beiden Antriebsarten - elektrischer Servomotor 30 und Handkurbel - gegeneinander ausgetauscht werden. Der Servomotor ist mit einem Untersetzungsgetriebe 31 versehen, dessen Getrieberad mit einem Wellenzapfen 32 drehmomentübertragend in eine Bohrung 33 der Antriebsschnecke 8 greift. Der Servomotor 30 ist mit dem Getriebe 31 halbseitig durch ein Gehäuse 34 umgeben. Es zeigt einen Befestigungsflansch 35, der mit Hilfe von Schrauben 36 an dem Gehäuse des Prüfgeräts befestigbar ist.

Alternativ hierzu greift eine von einer nicht dargestellten Handkurbel betätigbare Welle 40 in die Schnecke 8. In diesem Falle wird die Aufnahmebuchse 37 für das Untersetzungsgetriebe 31 durch einen Deckel 41 mit einer daran angeordneten Wellendichtung 42 verschlossen. Zur Befestigung dienen wieder die Befestigungsschrauben 36, mit denen auch die gehäuseförmige Motorabdeckung 34 mit dem Prüfgerät verbunden wird.

Der Servomotor 30 ist mit einer Vergleichs- und Regelelektronik ausgerüstet, die in Verbindung mit einem vom Motor angetriebenen Tachogenerator 45 einen Regelkreis bildet, durch welchen die Änderung der Motordrehzahlen feinfühlig in Abhängigkeit vom Rampenverlauf korrigiert werden können. Dieser Regelkreis enthält als elektronische Bausteine eine Meßbrücke 46, welche über einen Meßverstärker 47 mit einem Impedanzwandler 48 verbunden ist. An den Impedanzwandler schließt sich eine Anzeigeeinrichtung 49 an, mit der die momentane Belastung angezeigt wird. Der Impedanzwandler ist außerdem über einen weiteren Anschluß 50 mit einem Vergleicher 51 verbunden. Von diesem führt eine weitere Verbindungsleitung 52 zum Sollwertgeber (Rampe) 53. Der Vergleicher 51 kann nunmehr die über die Verbindung 50 erhaltenen Istwerte mit den Sollwerten der Leitung 52 vergleichen. Die durch eine elektronische Subtraktion erhaltene Regelgröße kann über die Verbindung 54 einem Leistungsverstärker und -vergleicher 55 zugeführt werden. Dieser erhält über eine Verbindung 56 vom Tachometergenerator 45 die für einen weiteren Vergleich mit der Regelgröße notwendigen Istwerte, so daß eine sehr trägheitsarme ständige Anpassung des Drehmomentverlaufs des Servomotors 30 an die Spannungskurve der Rampe erreicht wird.

Das Haftzugprüfgerät ist zusätzlich - was insbesondere für den Handkurbelbetrieb von Bedeutung ist - mit wenigstens drei optischen Anzeigevorrichtungen zur Anzeige der vorschriftsmäßigen Belastungsgeschwindigkeit, die beim Drehen kontinuierlich eingehalten werden muß, und für Abweichungen von der gewählten Belastungsgeschwindigkeit in den positiven und den negativen Bereich ausgerüstet. Vorzugsweise werden hierfür grüne, gelbe und rote Leuchtanzeigen (LED) eingesetzt. Die eine der Leuchtanzeigen (grün) leuchtet auf, sobald die Drehzahlen der Handkurbel und damit die Belastungsgeschwindigkeit richtig sind. Sobald die Drehgeschwindigkeit etwas nach unten oder oben von dem Sollwert abweicht, leuchtet eine andere Anzeige (gelb) auf. Erhebliche Abweichungen nach oben oder unten werden durch die dritte Leuchtanzeige (rot) angezeigt. Die Genauigkeit der Belastungszunahme ist dadurch selbst bei einem Handkurbelbetrieb sehr hoch.

## Ansprüche

1. Haftzugprüfgerät zur Ermittlung der Haftzugfestigkeit von Beton, Estrich, Mörtel, Bitumen und dergleichen sowie von Beschichtungen aus Natur- und Kunststoffen, bestehend aus einem Traggestell für einen Zugaufnehmer, an dessen dem Prüfobjekt zugewandten Ende ein Prüfstempel befestigt ist und mechanischen Antriebselementen zur Erzeugung der auf das zu prüfende Objekt zu übertragenden Zugkraft, dadurch gekennzeichnet, daß der Zugaufnehmer (5) über ein Schneckengetriebe (7, 8) mit einer von einem Netzanschluß unabhängigen Antriebsvorrichtung verbunden ist.

2. Haftzugprüfgerät nach Anspruch 1, dadurch gekennzeichnet, daß als Antrieb eine Handkurbel (40) vorgesehen ist.

3. Haftzugprüfgerät nach Anspruch 1, dadurch gekennzeichnet, daß als Antrieb ein von einem Akkumulator gespeister elektrischer Servomotor (30) eingesetzt ist.

4. Haftzugprüfgerät nach Anspruch 3, dadurch gekennzeichnet, daß der Servomotor (30) mit einem Untersetzungsgetriebe (31) als gegen die Handkurbel (40) austauschbare, von einem Gehäuse (34) halbseitig umschlossene Einheit an dem Prüfgerät befestigbar ist, wobei die Antriebswelle (32) des Getrieberades drehmomentübertragend in die Antriebsschnecke (8) greift.

5. Haftzugprüfgerät nach Anspruch 3 oder 4, dadurch gekennzeichnet, daß der Servomotor eine elektronische Regelschaltung aufweist.

6. Haftzugprüfgerät nach einem oder mehreren der Ansprüche 1 bis 5, dadurch gekennzeichnet, daß das Schneckenrad (7) mit einem Innengewinde in das Außengewinde einer hülsenförmigen Gewindespindel (6) greift und diese formschlüssig mit dem Zugaufnehmer (5) verbunden ist.

7. Haftzugprüfgerät nach Anspruch 6, dadurch gekennzeichnet, daß der Zugaufnehmer mit einem Flansch (5a) stirnseitig an der Gewindespindel (6) hängend befestigt ist.

8. Haftzugprüfgerät nach einem oder mehreren der Ansprüche 1 bis 7, dadurch gekennzeichnet, daß das Schneckenrad (7) über ein Drucklager (9) auf dem Traggestell (2) aufliegt.

9. Haftzugprüfgerät nach Anspruch 2, dadurch gekennzeichnet, daß die Handkurbel über einen Wellenzapfen (40) mit der in das Außengewinde des Schneckenrades (7) greifenden Antriebsschnecke (8) verbunden ist.

10. Haftzugprüfgerät nach Anspruch 4, dadurch gekennzeichnet, daß der Wellenzapfen (32) des Getrieberades formschlüssig in eine Aufnahmebohrung (33) der Antriebsschnecke (8) greift.

11. Haftzugprüfgerät nach einem oder mehreren der Ansprüche 1 bis 10, dadurch gekennzeichnet, daß der Zugaufnehmer (5) mit einer Meßbrük-ke aus Dehnungsmeßstreifen versehen ist, welche über einen Meßverstärker (47) und einen sich daran anschließenden Impedanzwandler (48) mit einer optischen Meßwertanzeige (49) verbunden ist.

12. Haftzugprüfgerät nach einem oder mehreren der Ansprüche 1 bis 11, dadurch gekennzeichnet, daß an den Servomotor (30) ein mit der Regel-schaltung verbundener Tachometergenerator ange-schlossen ist.

13. Haftzugprüfgerät nach einem oder mehreren der Ansprüche 1 bis 12, dadurch gekennzeichnet, daß der Vergleicher (51) der Regelschaltung mit dem Impedanzwandler (48) als Istwertgeber einerseits und mit dem Sollwertgeber (53) andererseits und der Ausgang des Vergleichers über einen Leistungsverstärker und -vergleicher (55) mit dem Servomotor verbunden ist.

14. Haftzugprüfgerät nach einem oder mehreren der Ansprüche 1 bis 13, dadurch gekennzeichnet, daß der Ausgang des Tachometers (45) als Istwertgeber mit dem gleichzeitig als Vergleicher wirkenden Leistungsverstärker (55) verknüpft ist.

15. Haftzugprüfgerät nach einem oder mehreren der Ansprüche 1 bis 14, dadurch gekennzeichnet, daß der Prüfstempel (25) über eine Schraubmutter (24) an einer Kugelkalotte (21) aufgehängt ist, welche ihrerseits mit einem drehbeweglich am Zugaufnehmer (5) befestigten Gelenkkopf (20) verschraubt ist.

16. Haftzugprüfgerät nach einem oder mehreren der Ansprüche 1 bis 15, dadurch gekennzeichent, daß der Gehäuseeinsatz (3a) an seinem oberen Ende mit einer Frontplatte (3b) verschlossen ist, auf welcher Leuchtdioden (60) zur Kontrolle der Belastungsgeschwindigkeit angeordnet sind.

Fig.1

Fig.2

Fig.3

EP 0 359 140 A2

Fig. 4

Fig.5

60

EP 0 359 140 A2